Europäisches Patentamt

European Patent Office

Office européen des brevets

(11) Publication number: **0 477 973 A1**

## EUROPEAN PATENT APPLICATION

(21) Application number: **91116562.9**

(22) Date of filing: **27.09.91**

(51) Int. Cl.⁵: **A61M 5/14**, A61M 5/172, G01L 1/22, G01F 1/00

(30) Priority: **28.09.90 US 589523**

(43) Date of publication of application:
**01.04.92 Bulletin 92/14**

(84) Designated Contracting States:
**DE FR IT**

(71) Applicant: **PALL CORPORATION**
**30 Sea Cliff Avenue**
**Glen Cove New York 11542(US)**

(72) Inventor: **Lipari, Charles P.**
**119 Nadia Court**
**Port Jefferson, New York 11777(US)**

(74) Representative: **Geissler, Bernhard, Dr. jur.,**
**Dipl.-Phys. Patent- und Rechtsanwälte et al**
**Bardehle-Pagenberg-Dost-**
**Altenburg-Frohwitter-Geissler & Partner**
**Postfach 86 06 20**
**W-8000 München 86(DE)**

(54) **Flow meter.**

(57) A flow meter provides a measurement of the rate of flow of a fluid into or out of a container (128) based on a measurement of the rate of change of the weight of the container (128). The weight of the container (128) exerts a load on a load cell (22) which deforms in accordance with the weight. Strain gauges mounted on surfaces of the load cell (22) and connected to form a bridge circuit vary in resistance according to the amount of deformation of the load cell (22). A differential voltage signal is produced at outputs of the bridge circuit. A differential amplifier is connected to receive the differential output from the bridge circuit and to output a signal related to the weight of the container (128). An analog differentiator receives the signal related to the weight and produces a signal related to the rate of change of the weight. The signal representing the rate of change is displayed as a measurement of the flow rate.

FIG. 4

FIG. 9

The present invention relates to an apparatus and method for measuring the rate of flow of a fluid and, particularly, for measuring the rate of flow by measuring the rate of change of weight of a container into or out of which the fluid is flowing.

In a wide range of situations, it is necessary to transfer fluid from a first container into a second container through an interconnecting duct. Typically, there is a desirable flow rate or range of flow rates for optimum system performance. Consequently, it may be desirable to measure and regulate the rate at which fluid flows in order to conform the actual flow rate to the desired flow rate or range of flow rates.

Measuring and regulating a fluid flow rate is often of particular importance in medical applications. Frequently, therapeutic compositions, such as nutrients or pharmaceuticals, and body fluids, such as blood or blood components, and so forth, are administered to a patient intravenously. In such a situation, rather than flowing from a first container to a second container, the fluids flow from a container directly into a patient's vascular system. Since these fluids must be administered at predetermined dosages for optimum therapeutic value, it is important that the fluid flow rate be monitored and regulated.

Typically, intravenous (IV) fluids are kept in containers, such as bottles or flexible bags, for easy storage and convenient administration. Fluids may be administered through flexible ducts or IV tubes which may be connected between the IV container and a needle or other suitable instrument for discharging the fluid into a vein of the patient. In such a system, it is necessary to measure the flow rate of the fluid accurately. A conventional flow meter system is known in which IV fluid is administered based on a volume displacement principle. A duct is connected between an IV fluid container and a patient. Disposed in the duct is an in-line pump which urges a known volume of fluids through a known length of duct with each cycle of operation. The fluid flow can then be regulated according to the number of cycles of pump operation per minute. Such systems are disadvantageously expensive.

A second conventional flow meter holds a container and uses a computer to control flow. The computer, which utilizes discrete-time, digital signals, operates a rotary pump head to deliver a predetermined amount of fluid per unit time, corresponding to a predetermined flow rate, from the IV container through suitable ducts. A flow meter as described has a disadvantageously high cost. This high cost is related to the cost of the components of the computer which may include a microprocessor and suitable support devices. Software or firmware for supporting the system and particularly for performing the required calculations according to the algorithms also involves a considerable cost in development time or license fees which must be amortized over the products sold.

The present invention provides a system for determining a rate of change of weight comprising: a weight transducer for carrying the weight and having a differential output for producing an output signal related to the weight; a differential amplifier having an input connected to the differential output and having an output for producing a first signal related to the weight; and an analog differentiator connected to the output of the differential amplifier to receive the first signal and having an output for producing a second signal proportional to the rate of change of the weight.

The present invention also provides a differential flow meter system for administering intravenous (IV) fluid to a patient at a predetermined rate comprising: a weight transducer having a carrier for supporting an IV container containing the fluid to be administered and a differential output for outputting a differential signal related to the weight of the IV container; and a circuit connected to the differential output and including a differential amplifier connected to receive the differential signal and to output a first signal proportional to the weight of the IV container and an analog differentiator connected to receive the first signal and to output a second signal proportional to the rate of change of the weight of the IV container.

The present invention further provides a method for determining the rate of change of the amount of fluid in a container comprising the steps of: placing the container on a weight transducer which produces a first analog signal related to the weight of the fluid in the container; differentiating the first analog signal to produce a second analog signal proportional to the rate of change of the weight of the first container; and displaying a numerical value related to the second signal.

The present invention additionally provides a method for administering intravenous (IV) fluid to a patient at a predetermined rate comprising the steps of: placing an IV container on a weight transducer; producing a differential output signal related to the weight of the IV container; supplying the differential output signal to a differential amplifier which outputs a first voltage related to the weight of the IV container; supplying the first voltage to an analog differentiator which outputs a second voltage proportional to the rate of change of the weight of the IV container; and supplying the second voltage to a display for displaying a numerical value related to the second voltage and to the rate of change of the weight of the IV container.

A system for determining a rate of change of a weight and a flow meter system for administering IV fluid in accordance with the invention utilizes analog hardware, such as an analog differentiator, to eliminate the need for expensive microprocessor software and expensive digital components. Similarly, the method for determining the rate of change of the amount of fluid in a container and the method for administering IV fluid produce signals related to the rate of change of weight without requiring the intervention of expensive digital hardware or complex, expensive digital software calculations. Accordingly, considerable savings are realized without sacrificing accuracy.

Figure 1 is a block diagram of a device for determining a rate of change of a weight and displaying the rate of change.

Figure 2 is a perspective view of the apparatus of Figure 1.

Figure 3 is a cut-away top view of the apparatus of Figure 2.

Figure 4 is a cut-away side view of the apparatus of Figure 2.

Figure 5 is a side view of the load cell shown in Figure 4 including schematic representations of electrical connections.

Figure 6 is a top view of a strain gauge mounted on the load cell of Figure 5.

Figure 7 is a side view of the load cell of Figure 5 showing physical deformation due to weight.

Figure 8 is a schematic representation of a circuit including a bridge circuit showing the strain gauges of Figure 5 and a differential amplifier/differentiator circuit as shown in Figure 1.

Figure 9 is a perspective view of a system for transferring fluid from a first container to a second container, the second container resting on an apparatus as shown in Figure 2.

Figure 10 is a perspective view of a system for administering intravenous fluid to a patient employing the apparatus of Figure 2.

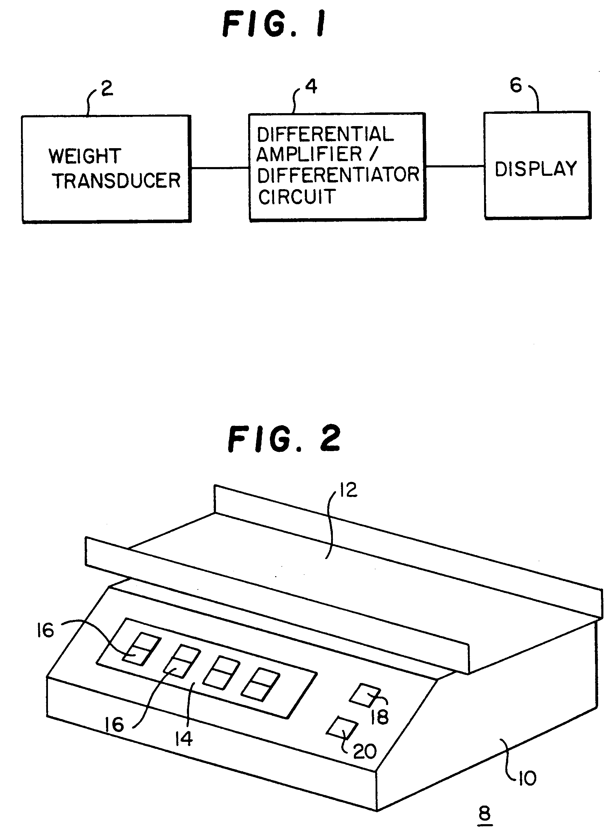

Figure 1 is a block diagram of an apparatus which generally comprises a weight transducer 2, a differential amplifier/differentiator circuit 4, and a display 6. The weight transducer 2 is a structure suitable for producing a signal related to a weight. In a preferred embodiment, the weight transducer 2 is a structure upon which the weight to be measured is placed. It may have a substantially flat upper surface on which the weight is placed, or it could include a tray or saddle for accommodating the weight. If a fluid container is the weight, then the rate of change of the weight is related to the flow rate.

Additionally, the weight transducer includes an output for outputting a continuous signal related to the weight. The output signal might be an electrical signal, such as a current or a voltage. Alternatively, the output signal could be related to a strain or physical displacement of all or part of the weight transducer in response to the weight. The signal could be proportional to the weight or have some other linear relationship, such as having a quiescent value corresponding to zero weight, the value deviating from the quiescent value in accordance with the weight.

In a preferred embodiment, the weight transducer includes a load cell. Load cells are commonly employed for producing an electrical signal related to a weight. A more detailed description of a preferred embodiment, including a description of a load cell employed as a weight transducer, may be found below.

The continuous signal produced by the weight transducer is provided via a suitable connection to the differential amplifier/differentiator circuit 4. A primary function of the circuit 4 is to produce a continuous signal related to the rate of change of the weight placed on the weight transducer 2. This may be accomplished by differentiating a continuous signal related to the magnitude of the weight. In a preferred embodiment, the circuit 4 includes an analog differentiator circuit. Such a circuit includes an operational amplifier having a suitable feedback network, such as an RC circuit or a tank circuit. The nature of the differentiator may be selected based on the anticipated rate of change of the weight. In an application such as a system for administering IV fluid to a patient, the weight is not likely to change rapidly. Accordingly, components for the differentiator may be selected which do not require rapid transition times.

As will be discussed in more detail below, the load cells may include a bridge circuit which produces a differential output. If the signal output from the weight transducer 2 and received by the circuit 4 is a differential signal, then it may be desirable to provide a differential amplifier as part of the circuit 4. Such a device might include an operational amplifier having a differential input, such as a non-inverting input and an inverting input. Appropriate circuitry produces a ground-reference continuous signal related to the weight on the weight transducer 2. This signal is then provided to the differentiator.

A continuous output signal related to the rate of change of the weight on the weight transducer 2 is then output from the circuit 4 to the display 6. The display may include any suitable apparatus for providing a quantitative measurement to a human operator. For instance, an A/D and a digital meter having a suitable number of display digits could be employed. Alternatively, an analog galvanometer-type display could be employed.

The objective of the display 6 is to provide the human operator with something he would understand to be a quantitative measurement of the rate of change of the weight. In a system for administering IV fluid to a patient, this may effectively be a quantitative measurement of the flow rate of the fluid from the IV container. Thus, it is desirable that the display 6 produce a number in units convenient for either weight or flow rate.

Since a standard voltmeter which might be employed as a display displays in units of volts, it is desirable for the signal value to have a magnitude such as one volt per gram/second of weight change or one volt per milliliter/minute of flow. Accordingly, it may be desirable to employ scaling circuitry which receives the rate of change signal from the circuit 4 and scales it to a suitable magnitude corresponding to convenient units for display, such as a flow rate in milliliters per minute or a rate of weight change in grams per second. An appropriate circuit, such as a linear amplifier, may be employed as an input stage to the display 6. Alternatively, the differentiator or differential amplifier could be implemented to have a gain suitable for scaling the respective signals to an appropriate amplitude.

It is anticipated that this type of system will be employed in a situation in which an optimal rate of weight change or flow rate or an optimal range of weight change rates or flow rates is desired. The human operator will have knowledge of such optimal values. He will periodically or occasionally compare the displayed value with the optimal value and make a determination as to whether the displayed value conforms to the desired value or values. If it does not, then the human operator will take appropriate action to change the rate of weight change or flow rate. If the rate of change of weight is due to fluid flow, the operator may employ an appropriate mechanism for regulating the flow rate. For example, if fluid is flowing into or out of a container through a duct, the operator may place a clamp on the duct, partly restricting passage through the duct. The degree of restriction may be varied to vary the flow rate and cause it to conform to the desired value.

Alternatively, fluid flow out of a container may be related to the height of the container relative to the destination of the fluid due to gravity. For instance, if IV fluid is being administered to a patient, an IV container, such as a bottle or flexible bag, may be suspended on a rack above the patient. In accordance with the invention, the rack may include an adjustable support for raising or lowering the container. In such a situation, the human operator can make the flow rate as indicated by the display conform with the desired flow rate by raising or lowering the IV container relative to the patient.

Similarly, fluid may be flowing from a first container to a second container, the first container being higher than the second container, the fluid flowing wholly or partly under the influence of gravity. Either one of the containers may be placed on an adjustable support. The container on the support may then be raised or lowered relative to the other container to change the flow rate to conform to the optimal flow rate.

Figure 2 shows a perspective view of an apparatus 8 for determining a flow rate and providing a numerical value related to the flow rate. The apparatus 8 includes a body 10 which houses components which will be described in more detail below. A carrier shown as a tray 12 is provided on the top of the apparatus 8 for holding the weight. A display 14 including a plurality of seven-segment display digits 16 is provided on a front panel of the apparatus 8. The front panel may also include other suitable functions, such as a power switch 18 and a reset button 20. Preferably, the apparatus 8 is portable and small enough to be conveniently employed in a crowded hospital setting for administering fluid to a patient. Also, the apparatus 8 should be suitably light-weight so that it can be placed on an adjustable support for an IV rack for convenient raising and lowering to regulate the fluid flow rate.

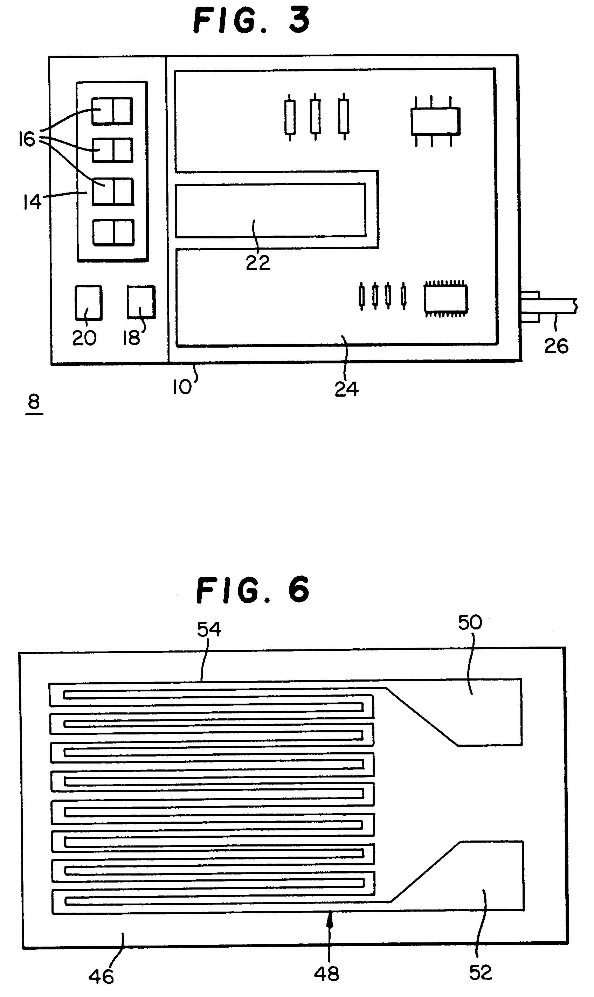

Turning to Figure 3, there is shown a top cutaway view of the apparatus of Figure 2. A load cell 22 is positioned beneath the tray 12, preferably near the center of gravity of the apparatus 8. The load cell forms a part of a weight transducer for converting a load acting on the load cell, such as a weight exerting a gravitational force on the load cell, into a signal related to the magnitude of the weight. In a preferred embodiment, a plurality of strain gauges are mounted on various surfaces of the load cell to make up the weight transducer. When a weight acts on the load cell, deformation of the load cell results, causing the strain gauges to experience a mechanical strain, i.e., a compression or stretching. Each of the strain gauges experiences different forms of strain according to factors such as which surface of the load cell the strain gauge is mounted on, how the strain gauge is oriented, the configuration of the load cell, and the magnitude of the weight.

A printed circuit board 24 carries electrical components required for implementing the differential amplifier/differentiator circuit 4. The board 24 is shown as having a U-shape for partially surrounding the load cell 22 and for lying parallel to the surface on which the apparatus 8 rests. Alternatively, the board could have any shape suitable for holding the required components and for being accommodated within the body 10. Also, the components could be disposed on a plurality of smaller boards connected by means of a, suitable back plane or a flexible bus, such as a ribbon cable having a series of edge connectors. An AC power cord 26 is provided for plugging the apparatus 8 into a standard wall socket. Suitable power supply components, such as a transformer, rectifier, and low-pass filter, not shown, may be provided for producing required DC power. Alternatively, the apparatus 8 could have a detachable input for a plurality of required DC voltage values. The input could be designed to accommodate a modular plug connected to a power supply assembly which plugs into a standard AC wall socket to generate DC power. In a preferred embodiment described in more detail below, +5v and -5v DC are required power supply voltages. Preferably, a regulated power supply which is isolated from the AC power line by means of a transformer is employed.

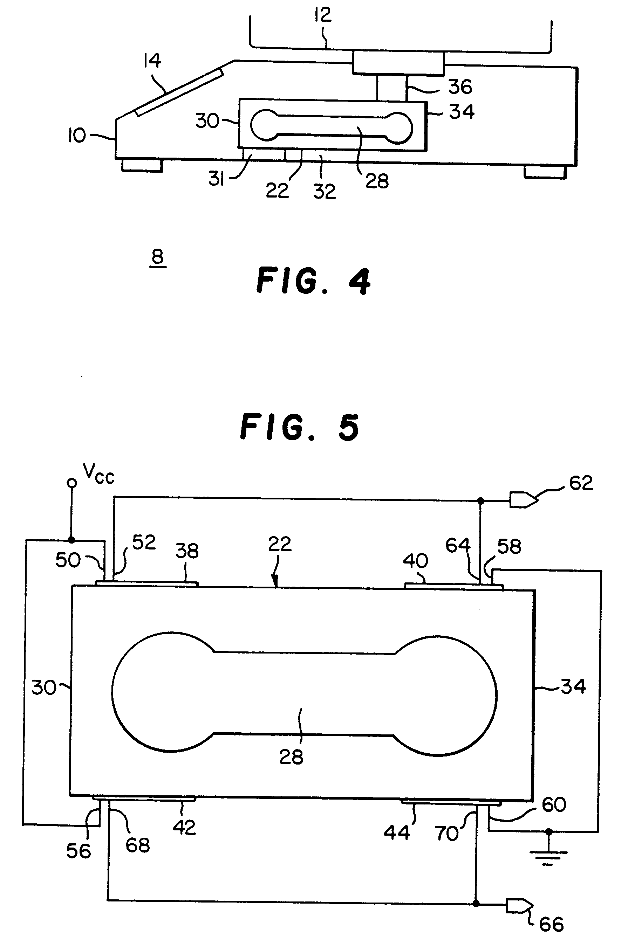

Figure 4 shows a side cut-away view of the apparatus 8. The load cell 22 may be in a variety of shapes, sizes, and configurations. In a preferred embodiment as shown in Figure 4, the load cell 22 may be in the shape of a rectangular prism having a dumbbell-shaped hole 28 passing through from side to side. The side view shown in Figure 4 provides a view directly through the hole 28 through the width of the load cell 22. The load cell 22 may be made of any substance which deforms under a desirable range of magnitudes of a load to a degree related to or, preferably, proportional to the magnitude of the load but which overcomes the deformation and returns to its original shape when the load is removed. In a preferred embodiment, the load cell 22 may be made of steel. The dimensions of the load cell illustrated in Figure 4 may be as follows: length (left to right) 7.42 cm (2.92 inches), height (top to bottom) 2.41 cm (.95 inch), thickness (perpendicular to page) 1.49 cm (.585 inch). The circular portions of the hole are 2.10 cm (.825 inch) in diameter. Their centers are 2.29 cm (.902 inch) from the respective ends of the load cell 22 and centered between the upper and lower surfaces of the load cell 22. The portion of the hole connecting the two circular portions is 8.25 mm (.325 inch) in height.

A load cell as described above has been rated at a maximum load of 1.6 kg (3.5 pounds). Within the range of 0-1.6 kg (0-3.5 pounds) load, measured strain has been found to change linearly with load changes to a precision of under one gram. By changing the dimensions given above, increasing or decreasing the overall size of the load cell, configuring the load cell differently (such as changing the shape of the hole), etc., it is possible to produce load cells having a wide variety of different load limits, sensitivity to load change, etc. The load limit, for instance, may be 225 kg (500 pounds) or higher.

The load cell 22 includes a first end 30 which is mounted to the bottom of the body 10 of the apparatus 8 by means of a suitable mounting member 31 to form a cantilever beam. Preferably, the mounting member 31 should have a thickness great enough to provide a gap 32 between the lower surface of the load cell 22 and the bottom of the body 10 large enough to prevent the lower surface of the load cell 22 from coming into contact with the bottom of the body 10 for any deformation within the load cell's rated range of loads.

The load cell 22 also has a second end 34. A mounting member 36 is mounted on the upper surface of the load cell 22 at the second end 34. The mounting member 36 runs upward through an upper portion of the body 10 to the exterior of the body 10. The tray 12 is permanently or detachably mounted to or placed on the mounting member 36 for carrying the weight.

Figure 5 provides a more detailed view of the load cell 22. The mounting members 31, 36 are not shown. Four strain gauges 38, 40, 42, 44 are disposed on upper and lower surfaces of the load cell 22 near the first and second ends 30, 34. Preferably, one of the four strain gauges is provided on either surface (upper, lower) of either end 30, 34 as shown.

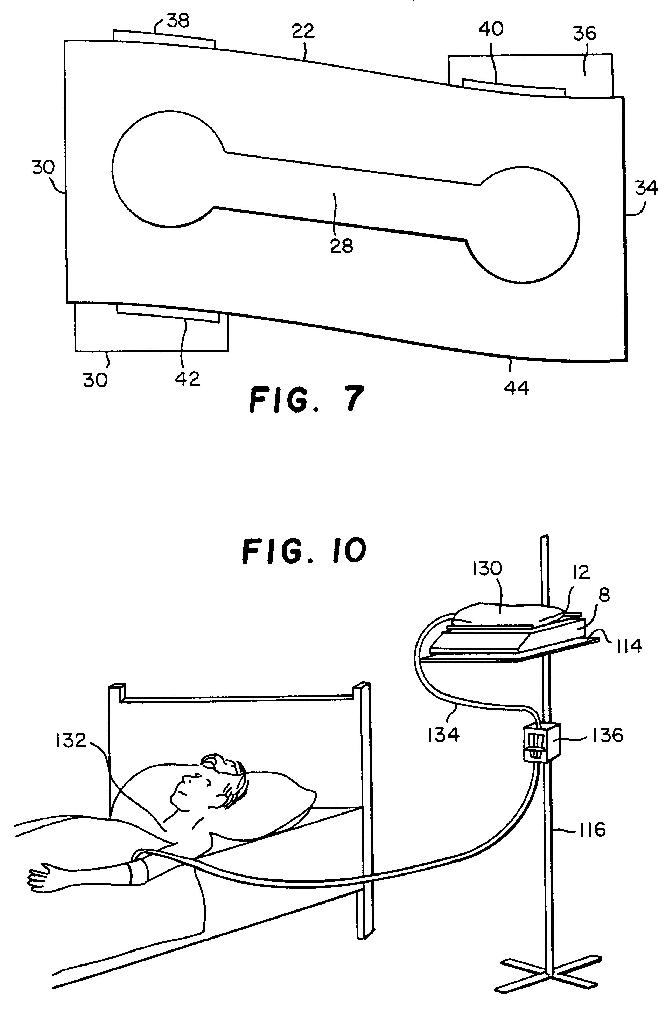

Figure 6 provides a more detailed illustration of one of the strain gauges according to a preferred embodiment. While the strain gauge of Figure 6 is identified as 38, it will be understood that the remaining strain gauges 40, 42, 44 of Figure 5 are substantially similar in overall structure, although the precise layout may vary.

The strain gauge 38 includes a substrate 46 which may be a printed circuit board, a film made of a substance such as mylar, or other suitable structure for carrying conductive elements of an electrical circuit. In the illustrated strain gauge 38, a metal foil conducting element is shown generally as 48. The element 48 may be a conductive alloy rolled to a thin foil, an element made by a photoresist/etching process, or other suitable conductive element. The conducting element 48 includes first and second contacts 50, 52. These contacts are suitable for making electrical connections using known techniques, such as soldering. The conducting element 48 also includes a series of grid lines 54. The grid lines 54 are shown as a conductor zig-zagging back and forth to provide a large total length between the contacts 50, 52. The width, length, and physical arrangement of the grid lines 54 are chosen so that there will be a finite predetermined electrical resistance between the contacts 50, 52. If the strain gauge 38 is subjected to physical strain, such as bending, causing the grid lines 54 to be stretched or compressed along their lengths, there will be a change in the value of the resistance measurable between the contacts 50, 52. The change in resistance may be due to piezoelectric or other effects, depending on the composition of the grid lines 54. In a preferred embodiment, stretching and compressing cause opposite changes in the resistance. For instance, stretching might cause a resistance increase and compressing, a decrease. The opposite may be true: stretching causing a decrease and compressing causing an increase. Which is true may depend on factors such as the composition of the element 48.

Referring to Figure 5, the strain gauges 38, 40, 42, 44 are disposed on the surfaces of the load cell 22 such that the grid lines 54 are parallel to the length of the load cell 22. Other configurations may also be employed as long as measurable resistance changes result from strain.

Electrical connections are shown in schematic form. A power supply voltage VCC is connected to the first contact 50 of the strain gauge 38 and to a first contact 56 of the strain gauge 42. Ground is connected to a first contact 58 of the strain gauge 40 and to a first contact 60 of the strain gauge 44. A first output 62 is connected to the second contact 52 of the strain gauge 38 and to a second contact 64 of the strain gauge 40. A second output 66 is connected to a second contact 68 of the strain gauge 42 and to a second contact 70 of the strain gauge 44. Accordingly, the strain gauges 38, 40, 42, 44 are wired together as a bridge circuit with two sets of the strain gauges 38, 40 and 42, 44 wired in series between VCC and ground. The bridge circuit has a differential output including the outputs 62, 66. An analog, i.e., continuous, voltage between the outputs 62, 66 varies as a weight causes a strain on the load cell 22 and deformation of the strain gauges. Changes in the resistances of the strain gauges 38, 40, 42, 44 caused by physical deformation of the load cell 22 may be detected with high accuracy based on the resultant voltage between the outputs 62, 66. In a preferred embodiment, the voltage between the outputs 62, 66 is related to the weight in the tray 12 of the apparatus 8 in which the load cell 22 is installed. As stated previously, the preferred load cell 22 deforms linearly with the load to within load changes of one gram or less over a rated load range of 0-1.6 kg (0-3.5 pounds).

Figure 7 shows the load cell 22 in a strained condition. Referring to Figure 4, it will be seen that, if a weight is placed in the tray 12, a downward force is exerted on the second end 34 of the load cell 22. The mounting member 31 provides support to the first end 30, but there is no comparable support beneath the second end 34. Accordingly, the second end 34 is displaced downward relative to the first end 30, and the shape of the load cell 22 is distorted as shown in Figure 7. The amount of distortion in the shape of the load cell 22 increases as the weight in the tray 12 increases. It will be seen that the strain gauges 40, 42 are bent in a concave manner, thereby compressing the grid lines 54. Similarly, the strain gauges 38, 44 are bent in a convex manner, thereby stretching the grid lines 54. The compression and stretching of the respective grid lines cause changes in the resistance values of the respective strain gauges. Thus, the differential voltage between the outputs 62, 66 reflects the strain exerted on the load cell 22 by the weight in the tray 12. The differential voltage may deviate from a quiescent value, such as zero volts, continuously and to a degree which increases as the weight increases. In a preferred embodiment of the flow meter, only a rate of change of weight is deemed significant. Accordingly, no offset adjustment is required for the strain gauges or the differential amplifier.

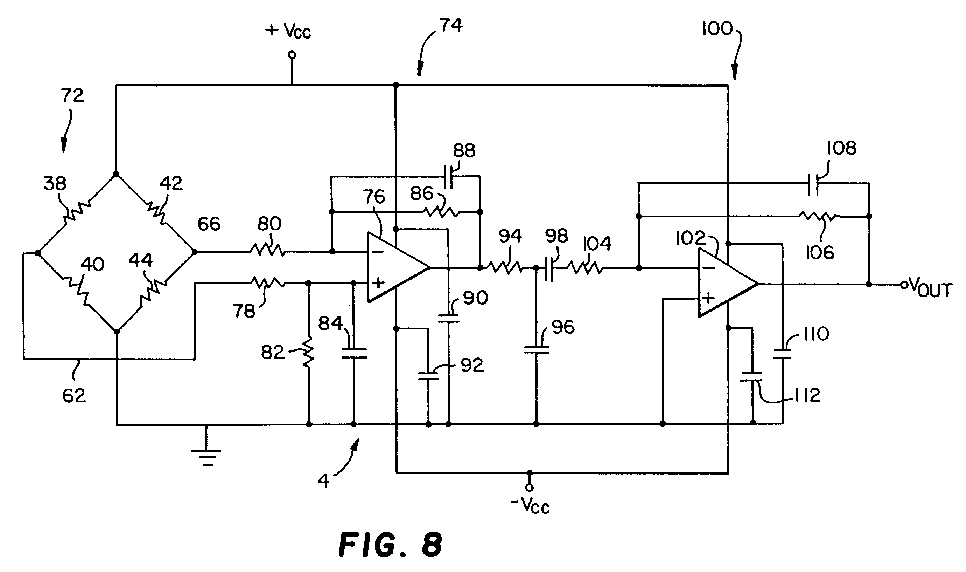

Figure 8 shows a detailed schematic of a preferred embodiment of the bridge circuit created by the strain gauges and the differential amplifier/differentiator circuit 4 as shown in Figure 1. Specifically, a bridge circuit 72 is shown including the strain gauges 38, 40, 42, 44 which are respectively shown as resistors.

The remainder of the components shown in the circuit of Figure 8 are included in the differential amplifier/differentiator circuit 4 of Figure 1. A differential amplifier 74 is shown comprising an operational amplifier 76 having positive and negative inputs connected respectively to the outputs 62, 66 of the bridge circuit 72 through resistors 78, 80, respectively. A resistor 82 and a capacitor 84 are connected in parallel between the positive input of the operational amplifier 76 and ground. The operational amplifier 76 has an output which is connected to the negative input through a resistor 86 and a capacitor 88 wired in parallel. Positive and negative power supply voltages +VCC, -VCC, are connected to power inputs of the operational amplifier 76. Power supply capacitors 90, 92 are connected between the respective power supply inputs and ground to maintain steady power supply voltage values. In a preferred embodiment, the differential amplifier 74 has a voltage gain of approximately 100.

A continuous signal which is related to the voltage differential between the outputs 62, 66 of the bridge circuit 72 and, hence, related to the weight, is output from the operational amplifier 76. This signal may vary slowly as the weight on the load cell 22 changes. The rate of change of the signal may be a constant, i.e.,

$$\text{rate of flow} = \frac{dV}{dt} = \text{constant}, \qquad (1)$$

where V is the signal output from the differential amplifier 74. To eliminate any high frequency noise, a low pass filter including a resistor 94 and a capacitor 96 are connected to the output of the operational amplifier 76. The voltage across the capacitor 96, which is the output of the low pass filter, then passes through a current isolation capacitor 98. Beyond the capacitor 98 is a differentiator 100.

The differentiator 100 includes an operational amplifier 102 having an inverting and a non-inverting input. The non-inverting input is connected to ground. The inverting input is connected through a resistor 104 to the capacitor 98. Accordingly, the output of the operational amplifier 76 of the differential amplifier is connected through the resistor 94, the capacitor 98, and the resistor 104 to the inverting input of the operational amplifier 102. An output of the operational amplifier 102 is connected to the inverting input through a feedback network including a resistor 106 and a capacitor 108 connected in parallel. Positive and negative power supply voltage inputs to the operational amplifier 102 are connected to ground through capacitors 110 and 112. A continuous output signal VOUT is output from the operational amplifier 102. The signal VOUT, having been differentiated, is proportional to the rate of change of the weight. The proportionality constant is related to the values of the components of the circuit, i.e.,

$$V_{out} = RC\frac{dV}{dt} \qquad (2)$$

where $V_{out}$ is the output voltage of the differentiator 100, R is the value of the resistor 106, C is the value of the capacitor 98, and dV/dt is the rate of change of the signal output from the operational amplifier 76 of the differential amplifier 74.

Equation (2) is derived as follows. A current I flowing through the capacitor 98 is

$$I = C\frac{dV}{dt} \qquad (3)$$

where C is the value of the capacitor 98 and dV/dt is the time derivative, i.e., rate of change, of the output voltage of the differential amplifier 74. The current I also flows through the resistor 106. The inverting input of the operational amplifier 102 is a virtual ground. The output voltage of the differentiator 100, $V_{out}$ is

$$V_{out} = IR \qquad (4)$$

where R is the value of the resistor 106. Substituting equation (3) for I in equation (4) yields equation (2). Accordingly, $V_{out}$ is a DC voltage proportional to the rate of change of V, the differential amplifier output voltage.

It should be noted that, in order for the above relationships to hold, the inverting input of the operational amplifier 102 should be protected from noise caused by stray electromagnetic fields, etc. In a preferred embodiment, the resistors 104 and 106 are mounted close to the operational amplifier 102. Also, the node where the resistors 104 and 106 meet the inverting input of the operational amplifier 102 is preferably surrounded by a grounded EM (electromagnetic) shield.

Upon initialization, the output voltage $V_{out}$ is preferably forced to zero and any initial charge on the capacitor 98 is removed. These initial conditions may be achieved by manually pressing the reset button 20, by means of a power-on reset circuit, or by other suitable means.

In summary, the circuit shown in Figure 8 produces a voltage signal proportional to a rate of change of a weight placed on the load cell 22 bearing the strain gauges 38, 40, 42, 44. The signal produced by the circuit represents the rate of change of the weight. If the weight is an IV container connected to a duct to permit fluid to flow in or out, then the rate of change in weight is related to the fluid flow rate into or out of the container. Accordingly, the output signal VOUT may be interpreted as a signal indicative of rate of change of weight or of a flow rate. The signal, which is proportional to the rate of weight change or of the flow rate, may be scaled as desired by means of an amplifier, voltage divider, or other suitable arrangement for producing a convenient relationship between units of measurement. For instance, by means of suitable scaling, a relationship could be established wherein a VOUT value of one volt corresponds with a flow rate of one milliliter per minute into the container. If suitable scaling is used, then a standard voltmeter or other electronic measuring device may be used to display a numerical value showing the desired flow rate or rate of change of weight in such convenient units.

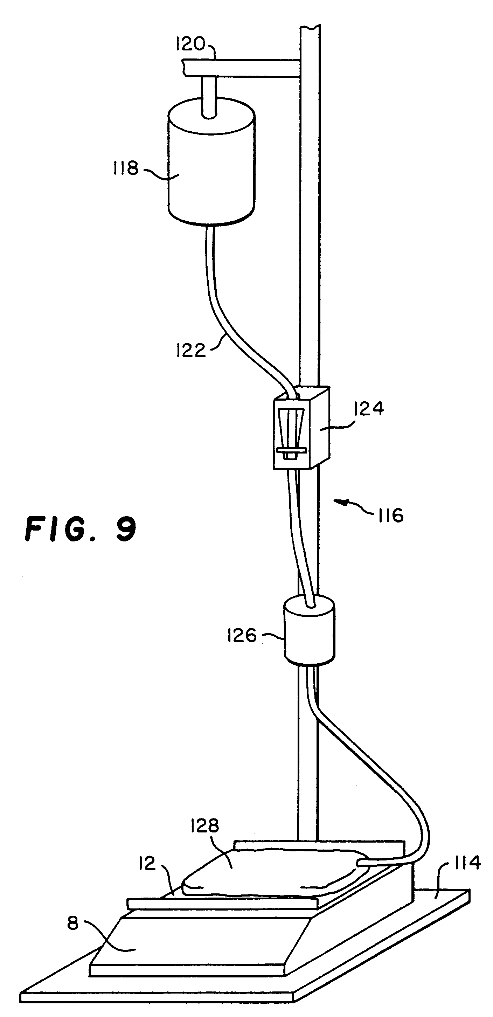

Figure 9 shows a system for transferring fluid between a first container and a second container employing an apparatus for measuring the rate of flow. An apparatus 8, as shown in Figure 2, is disposed on a support platform 114 of a stand 116. A first container 118 is suspended from an arm 120 of the stand 116. A duct 122 is connected to the first container 118 to conduct fluid from the container 118. The duct 122 runs to a fluid flow regulator 124. The regulator 124 may be any suitable apparatus for regulating fluid flow through the duct 122. For instance, it could include a clamp disposed around the duct 122 for pinching the duct 122 partially or entirely closed. The resulting decrease in the cross-sectional area of the interior of the duct 122 results in a restriction in fluid flow. Thus, in a preferred embodiment, the fluid flow regulator 124 is manually operated and the clamp preferably may be set to one of a range of settings, the setting causing varying degrees of pinching of the duct 122 and, hence, various degrees of restriction of fluid flow.

The duct 122 may continue to run to a filter 126. The filter may be any apparatus suitable for the particular type of fluid being conducted by the system. The filter 126 may include a membrane, a fibrous mass, or other suitable structures for removing particulate matter from the fluid. Also, the filter 126 may include an adsorption medium, such as activated carbon, for adsorbing chemical impurities from the fluid. If, for a given application, no such particulates or other impurities are present, the filter 126 may be omitted from the system.

The duct 122 continues to run to a second container 128. The second container 128 is shown resting on the tray 12 of the apparatus 8. In such an arrangement, the weight of the second container 128 is measured by the apparatus 8 and a rate of change of the weight of the second container 128 is displayed. In an arrangement as shown, the weight of the second container 128 increases as fluid flows into it. Thus, the displayed rate of change of weight may be interpreted as a flow rate into the container.

In a preferred embodiment, the height of the arm 120, the height of the support platform 114, or both, may be vertically adjustable on the stand 116. Accordingly, the height of the first container 118 relative to the second container 128 may be increased or decreased. The relative height may be changed in order to change the rate of flow of fluid from the first container 118 to the second container 128. If the flow rate is regulated in this manner, the fluid flow regulator 124 may be omitted from the system.

While Figure 9 shows an arrangement in which the first container 118 is suspended from the arm 120 above the second container 128, the second container 128 could be positioned above the first container 118. For example, the support platform 114 bearing the apparatus 8 could be positioned at a higher level than the arm 120. In such an alternative arrangement, fluid would flow from the container 128 on the support platform 114 to the container 118 suspended from the arm 120. In a preferred embodiment, the apparatus 8 is capable of measuring either a positive or negative rate of change of weight. In other words, fluid may flow either into or out of the container disposed on the tray 12.

A method for utilizing the apparatus of Figure 9 for determining the rate of change of the amount of fluid in a container may include the following steps. The container is placed on a weight transducer which produces a first analog signal related to the weight of the fluid in the container. In the embodiment shown in Figure 9, the weight transducer is shown as the load cell 22 incorporated into the apparatus 8. As described above, the bridge circuit illustrated in Figures 5 and 8 produces a continuous differential voltage signal related to the weight. Accordingly, since the weight transducer includes the load cell 22 and the strain gauges 38, 40, 42, 44 mounted on the load cell 22, it may be said that the weight transducer produces a first analog signal related to the weight of the container. The next step, which is performed by the differentiator 100, is differentiating the first analog signal to produce a second analog signal proportional to the rate of change of the weight of the container. Then, the display 6 displays a numerical value related to the second signal.

The method may additionally include the following steps performed by a human operator. First, the numerical value displayed on the display 6 is compared with a predetermined numerical value corresponding to a predetermined fluid transfer rate into or out of the container through the duct 122. Then, the flow of fluid through the duct 122 is regulated, thereby causing the displayed numerical value to conform to the predetermined numerical value. The step of regulating may preferably be made by a human operator. It may include adjusting the height of the containers relative to one another. Under these circumstances, the force of gravity may change the flow rate in accordance with the height differential between the two containers. Alternatively, the human operator might pinch the duct 122 using a clamp or other suitable mechanical device, thereby restricting the interior cross-sectional area of the duct 122 and restricting the flow of fluid. Also, in accordance with the use of an apparatus 8 including an analog differentiator 100, the step of differentiating may include performing an analog differentiation of the first signal.

Figure 10 shows a system for administering IV fluid to a patient at a predetermined rate. A flow meter apparatus 8, a stand 116, and a support platform 114 are substantially as shown in Figure 9. In the system of Figure 10, the support platform 114 is positioned at an elevation on the stand 116 to support the flow meter apparatus 8 and an IV fluid container 130 above a patient 132. A fluid duct, here shown as a flexible IV tube 134, is connected to conduct fluid from the container 130. The duct 134 is connected to a vein in an arm of the patient 132 by means of a needle or other suitable medical apparatus.

The duct 134 runs through jaws of a clamp 136 which may be employed for restricting the flow of fluid through the duct 134. The clamp 136 is here shown as being mounted on the stand 116. However, a clamp could also be freely disposed on the duct at any convenient position between the container 130 and the patient 132. For convenience of a human operator, the clamp 136 might preferably be close to the apparatus 8. Also, if the clamp is freely disposed on the duct 134, rather than mounted on the stand 116, it preferably should be lightweight and small in size.

The support platform 114 preferably is vertically adjustable along the stand 116. As an alternative to the clamp 136, the fluid flow rate may be regulated by adjusting the height of the support platform 114. Raising the support platform 114 increases the height of the container 130 relative to the patient 132, thereby increasing fluid flow rate. If the height of the support platform 114 is adjusted to regulate fluid flow rate, the clamp 136 may be omitted.

In the embodiments of Figures 9 and 10, the support platform 114 may be vertically adjusted along the stand 116 by any suitable means. For instance, a clamp structure on a side of the platform 114 abutting the stand 116 might be employed for clamping the support platform 114 securely onto the stand 116 at a desired height. Alternatively, the stand 116 might include a vertically arranged row of gear teeth or holes, and the support platform 114 might include a gear arrangement designed to mesh with the gear teeth or holes in the stand 116. In such an arrangement, the height of the support platform 114 might be adjusted by rotating a handle, causing a gear or other structure on the support platform 114 to rotate, thereby climbing up or down the stand 116. A suitable lock for securing the support platform 114 in place at a desired height may be disengaged by the operator before he rotates the handle to adjust the height and engaged thereafter.

The IV fluid administration system shown in Figure 10 might be employed as follows. An operator, such as a nurse or other medical personnel, places the IV container on the tray 12 of the apparatus 8. He then attaches the duct 134 to the container 130, attaches the other end of the duct 134 to a syringe or other suitable instrument and inserts the syringe or other instrument into the arm of the patient 132, thereby permitting IV fluid to flow into the patient's vein.

As fluid flows from the container 130 into the patient 132, the weight of the container 130 changes. As shown in Figure 8 and described above, the weight transducer, shown as the load cell 22 in Figure 3, is subjected to a mechanical strain due to the weight of the container 130. The bridge circuit 72 including the strain gauges 38, 40, 42, 44 on the load cell 22 produces a continuous differential output signal related to the weight of the IV container 130. The signal is supplied to the differential amplifier 74 which produces and outputs a first analog signal in the form of a first analog voltage related to the weight of the IV container 130. The first analog voltage is supplied to the analog differentiator 100 which outputs a second analog signal in the form of a second analog voltage proportional to the rate of change of the weight of the IV container 130. The second analog voltage is supplied to the display 6, and a numerical value related to the second analog voltage and to the rate of change of the weight of the IV container 130, i.e., related to the flow rate out of the container 130, is displayed on the display panel 14.

The nurse or other medical personnel periodically checks the numerical value and compares it with a predetermined numerical value related to a rate of change of weight which corresponds to a predetermined rate of administration of IV fluid determined in accordance with the patient's doctor's orders. The nurse manually regulates the fluid flow rate, either by employing the clamp 136 or by adjusting the height of the support platform 114 to cause the numerical value displayed on the display panel 14 to conform to the predetermined numerical value. Thus, by the foregoing method, the rate at which IV fluid is administered to the patient may be maintained at the value set by the patient's doctor's orders.

**Claims**

1. A system for determining a rate of change of a weight comprising:

   a weight transducer for carrying the weight and having a differential output for producing an output signal related to the weight;

   a differential amplifier having an input connected to the differential output and having an output for producing a first signal related to the weight; and

   an analog differentiator connected to the output of the differential amplifier to receive the first signal and having an output for producing a second signal proportional to the rate of change of the weight.

2. A system as recited in claim 1 wherein the weight transducer comprises a load cell and a bridge circuit including a plurality of strain gauges mounted on the load cell, the strain gauges having resistances which vary according to mechanical strain on the load cell imparted by the weight, the bridge circuit having the differential output.

3. A system as recited in claim 2 wherein the bridge circuit includes first and second strain gauges connected in series between a power source and ground, third and fourth strain gauges connected in series between the power source and ground, a first node between the first and second strain gauges, and a second node between the third and fourth strain gauges, and

wherein the differential output comprises the first and second nodes.

4. A system as recited in any one of claims 1-3 wherein the differential amplifier includes an operational amplifier circuit having an inverting input and a non-inverting input, the inputs being connected to the differential output of the weight transducer.

5. A system as recited in any one of claims 1-4 wherein the differentiator includes an operational amplifier circuit having a non-inverting input connected to ground, an inverting input connected to the output of the differential amplifier, an output, and a feedback circuit connected between the inverting input and the output.

6. A system as recited in claim 5 wherein the feedback circuit includes a resistor and a capacitor connected in parallel between the output and the inverting input.

7. A system as recited in any one of claims 1-6 further comprising a display connected to the output of the analog differentiator for receiving the second signal and for displaying a numerical value related to the rate of change of the weight.

8. A system as recited in claim 7 wherein the display includes scaling circuitry for converting the rate of change of the weight to a flow rate in convenient units.

9. A system as recited in claim 7 or 8 wherein the display includes a digital display.

10. A system as recited in claim 9 wherein the digital display includes a digital voltmeter.

11. A differential flow meter system for administering intravenous (IV) fluid to a patient at a predetermined rate comprising:

a weight transducer having a carrier for supporting an IV container containing the fluid to be administered and a differential output for outputting a differential signal related to the weight of the IV container; and

a circuit connected to the differential output and including a differential amplifier connected to receive the differential signal and to output a first signal proportional to the weight of the IV container and an analog differentiator connected to receive the first signal and to output a second signal proportional to the rate of change of the weight of the IV container.

12. A differential flow meter system as recited in claim 11 further comprising a display connected to receive the second signal for displaying a numerical value representing the rate of flow of the IV fluid.

13. A differential flow meter system as recited in claim 11 or 12 further comprising a stand having a vertically adjustable support for carrying the weight transducer at a desired height above the patient.

14. A differential flow meter system as recited in claim 13 wherein the height of the support on the stand is adjustable.

15. A differential flow meter system as recited in any one of claims 11-14 wherein the IV container is connected to the patient via an IV tube and wherein the differential flow meter system further comprises a clamp disposed on the IV tube for pinching the IV tube for regulating the rate of flow of fluid through the tube.

16. A method for determining the rate of change of the amount of fluid in a container comprising the steps of:

placing the container on a weight transducer which produces a first analog signal related to the weight of the fluid in the container;

differentiating the first analog signal to produce a second analog signal proportional to the rate of change of the weight of the first container;

and

displaying a numerical value related to the second signal.

17. A method as recited in claim 16 further comprising the steps of:

comparing the displayed numerical value with a predetermined numerical value corresponding to the predetermined fluid transfer rate; and

regulating the flow of fluid through the duct, thereby causing the displayed numerical value to conform to the predetermined numerical value.

18. A method as recited in claim 17 wherein the fluid flows between the container and a second container and the step of regulating includes adjusting the relative heights of the containers.

**19.** A method as recited in claim 17 or 18 wherein the step of regulating includes pinching a duct coupled to the container.

**20.** A method as recited in any one of claims 16-19 wherein the step of differentiating is analog differentiating.

**21.** A method for administering intravenous (IV) fluid to a patient at a predetermined rate comprising the steps of:

placing an IV container on a weight transducer;

producing a differential output signal related to the weight of the IV container;

supplying the differential output signal to a differential amplifier which outputs a first voltage related to the weight of the IV container;

supplying the first voltage to an analog differentiator which outputs a second voltage proportional to the rate of change of the weight of the IV container; and

supplying the second voltage to a display for displaying a numerical value related to the second voltage and to the rate of change of the weight of the IV container.

**22.** The method as recited in claim 21 further comprising:

comparing the displayed numerical value with a predetermined numerical value related to a rate of change of weight corresponding to the predetermined rate of administration of IV fluid; and

regulating the fluid flow rate responsive to the displayed numerical value to cause the displayed numerical value to conform to the predetermined numerical value.

**23.** A method as recited in claim 22 wherein the step of regulating includes adjusting the height of the IV container relative to the patient.

# FIG. 1

WEIGHT TRANSDUCER 2 → DIFFERENTIAL AMPLIFIER / DIFFERENTIATOR CIRCUIT 4 → DISPLAY 6

# FIG. 2

# FIG. 3

16

14

20  18

8

10  24

22

26

# FIG. 6

54  50

46  48  52

**FIG. 4**

8

**FIG. 5**

**FIG. 7**

**FIG. 10**

15

FIG. 8

FIG. 9

## DOCUMENTS CONSIDERED TO BE RELEVANT

EP 91116562.9

| Category | Citation of document with indication, where appropriate, of relevant passages | Relevant to claim | CLASSIFICATION OF THE APPLICATION (Int. Cl.5) |
|---|---|---|---|
| A | <u>CH - A - 600 892</u><br>(MEDIZENCO)<br>   * Fig. 2-6; claim; subclaims<br>     1,2,4; column 1, line 66 -<br>     column 2, line 4; column 2,<br>     lines 16-28; column 2, line<br>     65 - column 3, line 2 * | 1-4,<br>11,13,<br>16,17 | A 61 M 5/14<br>A 61 M 5/172<br>G 01 L 1/22<br>G 01 F 1/00 |
| A | <u>EP - A - 0 111 918</u><br>(KRÄMER)<br>   * Abstract; fig. 1,4; page 2,<br>     lines 18-27; page 3, lines<br>     10-14,19-26; claims 2,3,4 * | 1,2,<br>13,17 | |
| A | <u>FR - A - 1 552 266</u><br>(SIMONI)<br>   * Column 1, line 29 - column<br>     2, line 3; column 2, lines<br>     19-21; fig. 1; resume A * | 1,13,<br>17 | |
| A | <u>EP - A - 0 164 862</u><br>(TOKYO ELECTRIC)<br>   * Abstract; fig. 1,2; page 1,<br>     lines 1-5; page 5, lines<br>     1-5; claims 1-3,6 * | 2-4 | TECHNICAL FIELDS SEARCHED (Int. Cl.5)<br><br>A 61 M<br>G 01 L<br>G 01 F |
| A | <u>EP - A - 0 227 850</u><br>(HOTTINGER)<br>   * Abstract; fig. 1,3,7 * | 2 | |

The present search report has been drawn up for all claims

| Place of search | Date of completion of the search | Examiner |
|---|---|---|
| VIENNA | 10-01-1992 | ZAWODSKY |

EPO FORM 1503 03.82 (P0401)